Europäisches Patentamt

(19) European Patent Office

Office européen des brevets

(11) Veröffentlichungsnummer: **0 076 507**
**B1**

(12) **EUROPÄISCHE PATENTSCHRIFT**

(45) Veröffentlichungstag der Patentschrift:
**22.04.87**

(21) Anmeldenummer: **82109157.6**

(22) Anmeldetag: **04.10.82**

(51) Int. Cl.⁴: **A 61 B 10/00**

(54) **Gefäss zur Handhabung von pastösem Probenmaterial.**

(30) Priorität: **06.10.81 DE 3139702**

(43) Veröffentlichungstag der Anmeldung:
**13.04.83 Patentblatt 83/15**

(45) Bekanntmachung des Hinweises auf die Patenterteilung:
**22.04.87 Patentblatt 87/17**

(84) Benannte Vertragsstaaten:
**AT BE CH DE FR GB IT LI LU NL SE**

(56) Entgegenhaltungen:
**DE - B - 2 835 358**
**US - A - 4 293 405**

(73) Patentinhaber: **BOEHRINGER MANNHEIM GMBH,**
**Patentabteilung, Abt. E Sandhofer**
**Strasse 112-132 Postfach 31 01 20,**
**D-6800 Mannheim 31 Waldhof (DE)**

(72) Erfinder: **Kaspar, Klaus Peter, Dr., Gröberweg 1,**
**D-8132 Tutzing (DE)**
Erfinder: **Becker, Jürgen, Loisachstrasse 27,**
**D-8122 Penzberg-Maxkron (DE)**
Erfinder: **Huber, Marion, Amseiweg 8,**
**D-7411 Kleinengstingen (DE)**

(74) Vertreter: **Weickmann, Heinrich, Dipl.-Ing. et al,**
**Patentanwälte Dipl.-Ing. H.Weickmann Dipl.-Phys.Dr.**
**K.Fincke Dipl.-Ing. F.A.Weickmann Dipl.-Chem. B. Huber**
**Dr.-Ing. H. Liska Dipl.-Phys.Dr. J. Prechtel**
**Postfach 860820, D-8000 München 86 (DE)**

## Beschreibung

Die Erfindung betrifft ein Gefäss zur Handhabung von pastösem Probenmaterial mit einem ggf. röhrchenförmigen Gefässteil und einem auf das Gefässteil aufgesteckten, einen ggf. als Homogenisierungsraum dienenden Gefässinnenraum abschliessenden Deckelteil, wobei an der dem Gefässinnenraum zugewandten Deckelteilinnenseite ein ggf. zylindrischer Vorsprung im wesentlichen senkrecht absteht, an dessen freiem Ende stirnseitig eine zum Gefässinnenraum offene Probenausnehmung zur Aufnahme des Probenmaterials ausgebildet ist, deren Volumen dem gewünschten Probenvolumen entspricht.

Bei einem aus der DE-AS 2 835 358 bekannten Gefäss dieser Art wird die Probendosierung in der folgenden Weise vorgenommen:

Mit einem Spatel wird das Probenmaterial in die Probenausnehmung eingefüllt und anschliessend die eingefüllte Probe durch bündiges Entlangstreichen des Spatels entlang des Randes der Probenausnehmung glatt gestrichen. Man erhält so ein definiertes Probenvolumen, was für sich anschliessende quantitative Untersuchungsmethoden von wesentlicher Bedeutung ist. Die bei diesem Glattstreichen am Umfang des Vorsprunges im Bereich des Randes der Probenausnehmung sich zumeist anlagernden Probenbestandteile dürfen nicht in den Gefässinnenraum gelangen, da diese die anschliessenden quantitativen Untersuchungen verfälschen, insbesondere dann, wenn das im Gefässinnenraum vorhandene Probenmaterial zur Vorbereitung der quantitativen Messung in Lösungsmittel gelöst wird, welches in den als Homogenisierungsraum dienenden, zusätzlich zur Probenausnehmung vorgesehenen Gefässinnenraum zugegeben wird. Bei dem bekannten Gefäss wird das am Aussenumfang des Vorsprungs angelagerte überschüssige Probenmaterial manuell weggewischt. Dieser zusätzliche Arbeitsgang stellt im Falle unästhetischer Probenmaterialien, wie z.B. Stuhl, eine Belastung für die Dosierung vornehmende Person, insbesondere den Patienten, dar.

Demgegenüber ist es Aufgabe der Erfindung ein Gefäss eingangs genannter Art bereitzustellen, welches eine genaue Probendosierung bei einfacher, sauberer Handhabung erlaubt.

Diese Aufgabe wird dadurch gelöst, dass das Gefässteil im Bereich seiner Öffnung für das Deckelteil mit einem an dem Querschnitt des Vorsprungs angepassten Abstreifkragen für am Umfang des Vorsprungs angelagertes Probenmaterial ausgebildet ist, und dass auf der vom Gefässinnenraum abgelegenen Seite des Abstreifkragens im Deckelteil und/oder im Gefässteil ein Ringraum zur Aufnahme abgestreiften Probenmaterials ausgebildet ist. Zur Probendosierung muss demnach lediglich das Probenmaterial in die Probenausnehmung eingefüllt und anschliessend glatt gestrichen werden; anschliessend kann das Deckelteil sogleich auf das Gefässteil aufgesetzt werden, wobei der Abstreifkragen zwangsläufig dafür sorgt, dass überschüssiges Probenmaterial am Umfang des Vorsprungs abgestreift und in den Ringraum ausserhalb des Gefässinnenraums abgelagert wird. Das ggf. äusserst unangenehme manuelle Reinigen des Umfangs des Vorsprungs entfällt ganz.

Aus der US-PS 4 092 120 ist eine Einrichtung zum Dosieren und untersuchen von Stuhlproben bekannt, bei welchem die Stirnfläche eines Vorsprungs des Deckelteils 4 eben ausgebildet ist, also keine Probenausnehmung zur Aufnahme des Probenmaterials, deren Volumen dem gewünschten Probenvolumen entspricht, aufweist. Die Probendosierung wird bei dieser bekannten Einrichtung dadurch vorgenommen, dass in einen den Gefässinnenraum 8 definierenden, nach oben hin offenen Zylinderring Probenmaterial eingefüllt wird, woraufhin durch Schliessen des Deckelteils 4 der Vorsprung in den Zylinderring eindringt, wobei nach oben hin offene, axial verlaufende Schlitze 13 des Zylinderrings anfänglich ein seitliches Herausquellen überschüssigen Probenmaterials erlauben. Sobald die ebene Stirnfläche des Vorsprungs die unteren Enden der Schlitze 13 passiert hat, schliesst der Vorsprung mit dem Zylinderring nach Art eines Kolbens einigermassen dicht ab, wodurch das dosierte Probenvolumen festgelegt wird. Das Deckelteil 4 wird noch weiter auf das Gefässteil 1 zu bewegt, was zur Folge hat, dass das Probenmaterial auf ein unterhalb des Zylinderrings befindliches Filterpapier gepresst wird, wodurch flüssige Anteile des Probenmaterials für die anschliessende Untersuchung gewonnen werden könne. Erfindungsgemäss erfolgt die Probendosierung bereits vor dem Aufsetzen des Deckelteils auf das Gefässteil, nämlich durch Einfüllen der Probe in die Probenausnehmung innerhalb des Deckelteils und mit anschliessendem Glattstreichen. Diese Art der Dosierung ist zumal bei grösserem Vorsprungsdurchmesser wesentlich genauer, da bei der bekannten Lösung die tatsächliche Probendosis von mehreren, schlecht zu kontrollierenden Faktoren abhängt, wie z.B. von der Viskosität des Probenmaterials, und von den Abmessungen und gegenseitigen Zuordnungen dreier Teile im Augenblick des Schliessens der Schlitze 13 durch den Vorsprung nämlich der Teile Zylinderring mit Schlitzen 13, Vorsprung und gegenüberliegendes Filterpapier bzw. unteres Deckelteil 6. Schliesslich kommt es bei der Erfindung in erster Linie auf die Gewinnung der festen Anteile des Probenmaterials an; der bei geschlossenem Deckelteil zusätzlich zur Probenausnehmung vorgesehene Gefässinnenraum dient ggf. als Homogenisierungsraum für das in der Probenausnehmung des Deckelteils genau dosierte Probenmaterial. Hierzu ist das erfindungsgemässe röhrchenförmige Gerät zur Zentrifugierung eingerichtet.

Um ein Ausfliessen des abgestreiften Probenmaterials aus dem Ringraum nach aussen sowie ggf. eine entsprechende Geruchsbelästigung zuverlässig zu verhindern, wird vorgeschlagen, dass das Deckelteil und das Gefässteil mit einer den Ringraum nach aussen hin abdichtenden Umfangsdichtung ausgebildet sind, vorzugsweise in Form eines an der Deckelteilinnenseite vorstehenden Umfangsbundes, der mit einer Innenumfangsfläche an einer Aussenumfangsfläche einer Umfangsschulter des Gefässteils abdichtend anliegt.

Um ein sicheres Abstreifen überschüssigen Probenmaterials auch dann zu gewährleisten, wenn das Deckelteil versehentlich nicht vollständig auf das Gefässteil aufgeschoben wird, wird vorgeschlagen, dass der Vorsprung über den Umfangsbund vorsteht, vorzugsweise 1 bis 5 mm, am besten etwa 2 mm. Ferner wird vorgeschlagen, dass das Gefässteil mit einer mit dem Gefässinnenraum verbundenen weiteren Öffnung versehen ist, die mit einem wieder entfernbaren Zusatzdeckelteil verschlossen ist. Dies ermöglicht es, in dem Gefässinnenraum Zusatzmittel, wie z.B. Lösungsmittel oder Homogenisierungselemente, einzufüllen, ohne das Deckelteil lösen zu müssen, welches ggf. unästhetisches Probenmaterial trägt. Hierdurch wird die Belästigung des Laborpersonals bei der Vorbereitung der Untersuchungsmessungen des Probenmaterials wesentlich herabgesetzt.

Eine einfach und kostengünstig herstellbare Ausführungsform des Gefässes ist dadurch gekennzeichnet, dass das röhrchenförmige Gefässteil an einem seiner Rohrenden mit dem Deckelteil und am anderen Rohrende mit dem Zusatzdeckelteil verschlossen ist. Von Vorteil ist zudem, dass sich das röhrchenförmige Gefäss in üblicher Weise auf dem Postwege versenden lässt.

Bevorzugt ist vorgesehen, dass das Deckelteil und/oder das Zusatzdeckelteil je über eine Verbindungslasche vorzugsweise einstückig mit dem Gefässteil verbunden sind. Deckelteil und Zusatzdeckelteil sind demnach unverlierbar mit dem Gefässteil verbunden. Von besonderem Vorteil ist auch, dass nunmehr ein einziges Teil, insbesondere Kunststoff-Spritzgussteil, herzustellen ist, was die Herstellungskosten stark verringert.

Um die Handhabung des Deckelteils bei der Probendosierung bzw. das Lösen des Zusatzdeckelteils zu erleichtern, wird vorgeschlagen, dass das Deckelteil und/oder das Zusatzdeckelteil mit einer Grifflasche, vorzugsweise einstückig, verbunden sind.

Das Gefässteil kann neben der Probendosierung und dem Probentransport u.a. auch zum Zentrifugieren des im Gefässinnenraums in Lösung befindlichen Probenmaterials eingesetzt werden, wenn das Gefäss derart ausgebildet ist, dass das Gefässteil und das Deckel- und/oder das Zusatzdeckelteil einen Aussendurchmesser von ca. 17 mm aufweisen, und das ggf. die Verbindungs- und Grifflaschen vorzugsweise über Sollbruchstellen leicht abtrennbar sind. Die handelsüblichen Zentrifugen haben Aufnahmebohrungen von ca. 18 mm Innendurchmesser und sind daher zur Aufnahme der erfindungsgemäss ausgebildeten Gefässe geeignet. Über den Aussendurchmesser des Gefässes von ca. 17 mm hinausragende Verbindungs- oder Grifflaschen können aufgrund der Sollbruchstellen leicht abgetrennt werden.

Aus der bereits genannten DE-AS 2 835 358 ist es bekannt, in den Gefässinnenraum eine als Homogenisierungselement dienende Glaskugel einzusetzen, die zu einer Vermengung und Zerkleinerung des Probenmaterials mit einer Lösungsflüssigkeit (Extraktionsflüssigkeit) dient. Hierzu wird das Gefäss in einen Schüttler eingesetzt und durchgeschüttelt, was zu einer entsprechenden Hin- und Herbewegung der Glaskugel im Gefässinnenraum führt. Dabei stösst die Glaskugel hier und da an dem Aussenrand der Probenausnehmung und wird dadurch gehindert, in die Probenausnehmung einzudringen, was das Loslösen von Probenmaterial aus der Probenausnehmung insgesamt verschlechtert. Dies gilt insbesondere dann, wenn Probenmengen dosiert werden sollen, deren Volumen klein ist gegenüber dem Gefässinnenraumvolumen. In diesem Falle ist nämlich der Probenausnehmungsdurchmesser im allgemeinen wesentlich kleiner als die entsprechende Dimension des Gefässinnenraums. Um zu erreichen, dass das oder die in den Gefässinnenraum eingesetzten Homogenisierungselemente (Glas- oder Stahlkugeln, Stäbchen o.dgl.) bei der Schüttelbewegung stets in die Probenausnehmung gelangen, was ein schnelles, vollständiges Herauslösen von Probenmaterial aus der Probenausnehmung sicherstellt, wird vorgeschlagen, dass die den Gefässinnenraum begrenzende Gefässteilinnenfläche zur Probenausnehmung im Deckelteil hin vorzugsweise konisch zuläuft.

Beim Glattstreichen der Probe kann es gelegentlich passieren, dass auf dem Rand der Probenausnehmung an der Stirnseite des Vorsprungs noch überschüssiges Probenmaterial versehentlich hängenbleibt. Um zu verhindern, dass auch dieses überschüssige Probenmaterial vom Lösungsmittel im Gefässinnenraum mit aufgelöst wird und dadurch die quantitative Messung verfälscht, wird vorgeschlagen, dass das Gefässteil mit einem vorzugsweise hinterschnittenen Innenumfangsbund an der Stirnseite des Vorsprungs im Bereich des Randes der Probenausnehmung anliegt. Das überschüssige Probenmaterial wird folglich zwischen dem Innenumfangsbund und der Stirnseite des Vorsprungs festgehalten. Im Falle der bevorzugten Hinterschneidung des Innenumfangsbundes können auch relativ grosse überschüssige Probenmengen zuverlässig daran gehindert werden, in den Gefässinnenraum zu gelangen; bei an der Stirnseite des Vorsprungs anliegendem Innenumfangsbund bildet sich ein nach innen abgedichteter Ringraum zur Aufnahme überschüssigen Probenmaterials, der von der Hinterschneidung des Innenumfangsbundes und der die Probenausnehmung umgebenden, ggf. kreisringförmigen Stirnfläche des Vorsprungs begrenzt wird.

Eine bevorzugte Ausführungsform der Erfindung ist gekennzeichnet durch einen in den Gefässinnenraum einsetzbaren, spiralig ausgebildeten Homogenisierungsstab, dessen Spiraldurchmesser an dem Gefässinnendurchmesser angepasst ist. Der Homogenisierungsstab sorgt für eine intensive Zerkleinerung und Vermengung des Probenmaterials mit der Lösung, da er aufgrund seiner spiraligen Ausbildung bei seiner Hin- und Herbewegung innerhalb des Gefässinnenraums um seine Längsachse rotiert. Hierbei ist vorzugsweise vorgesehen, dass das der Probenausnehmung nahe Stabende des Homogenisierungsstabs sich im wesentlichen in Richtung der Spiralachse erstreckt, vorzugsweise mit Abstand zur Spiralachse, was ein vollständiges Herauslösen des Probenmaterials auch aus ggf. vorhandenen Eckbereichen am Boden der Probenausnehmung sicherstellt. Der Homogenisierungsstab gewährleistet demnach eine vollständige Auflösung des Probenmaterials, wobei auch grössere Probenteilchen fein zerschlagen werden. Bei den bekannten zur Homogenisierung eingesetzten Glaskugeln dagegen besteht zum einen die Gefahr, dass zumindest ein Teil des Probenmaterials nicht aus der Probenausnehmung herausgelöst sondern im Gegenteil durch die Kugeln an den Boden der Probenausnehmung festgedrückt wird; zum anderen ist die Zerkleinerungswirkung der Glaskugeln häufig nicht ausreichend.

Um zu erreichen, dass der Vorsprung des Deckelteils beim Schliessen des Deckels anfänglich leichtgängig in die Öffnung gedrückt und dennoch bei vollständig geschlossenem Deckelteil zuverlässig in der Öffnung hält, wird vorgeschlagen, dass der Vorsprung in Richtung zur Probenausnehmung leicht konisch zuläuft. Im Gefässteil ist vorzugsweise ein an den Querschnitt des Vorsprungs angepasster Aufnahmeraum für den Vorsprung vorgesehen, an dessen Vorsprung-Einsteckende der Abstreifkragen angeordnet ist und an dessen gegenüberliegendem, in den Gefässinnenraum übergehenden Ende, ggf. der hinterschnittene Innenumfangsbund, angeordnet ist. Bei zylindrischer Ausbildung dieses Aufnahmeraums wird beim Einstecken des Vorsprungs am Ende der Bewegung die den Aufnahmeraum umgebende, vorzugsweise aus Kunststoff bestehende Gefässwand elastisch verformt, was den erwünschten, festen Zusammenhalt zwischen Vorsprung und Gefässteil ergibt. Die gleiche Wirkung erzielt man, wenn der Aufnahmeraum auf den Gefässinnenraum leicht konisch zulaufend ausgebildet ist, wobei in diesem Falle der Vorsprung zylindrischen Querschnitt aufweist. Die konische Ausbildung von Vorsprung oder Aufnahmeraum erleichtert zudem das Einfädeln des Vorsprungs in die Gehäuseöffnung. Dabei ist die Konizität derart gering festgelegt, dass der Abstreifeffekt im wesentlichen ungeändert bleibt.

In einer Weiterbildung der Erfindung wird vorgeschlagen, dass der Umfangsbund an der Umfangsschulter des Gefässteils mittels einer Schnappverbindung festlegbar ist, vorzugsweise in Form wenigstens einer an einem der beiden Teile vorgesehenen Umfangserhebung, welche in eine am jeweils anderen Teil vorgesehene Umfangsvertiefung eingreift. Hierdurch erreicht man zum einen eine Verbesserung der Abdichtung des Ringraums nach aussen, was Geruchsbelästigungen durch im Ringraum eingeschlossenes überschüssiges Probenmaterial ausschliesst; zum anderen wird der Halt des geschlossenen Deckelteils am Gefässteil noch weiter verbessert, so dass ein unbeabsichtigtes Lösen des Deckels, z.B. während des Transports, ausgeschlossen ist.

Die Erfindung betrifft auch eine Verwendung des erfindungsgemässen Gefässes zur Enzymbestimmung, vorzugsweise Chymotrypsin- oder Trypsinbestimmung im Stuhl, insbesondere zur Stuhlprobendosierung von Mengen kleiner 1 g Stuhl, vorzugsweise zwischen 20 und 200 mg Stuhl. Das erfindungsgemässe Gefäss ist deshalb hierfür besonders gut geeignet, weil zum einen die Handhabung des Gefässes unter geringstmöglicher Belastung von Patient und Laborpersonal erfolgt und zum anderen deshalb, weil eine gut reproduzierbare Dosierung vergleichsweise kleiner Stuhlproben möglich ist; die vorgesehene photometrische Aktivitätsbestimmung ist bei grösseren Probenmengen aufgrund der Eigenfärbung der Probenlösung nicht mehr möglich. Da bei den bekannten Gefässen nur grössere Probenmengen ausreichend genau dosierbar sind, war es bisher nicht möglich, photometrische Aktivitätsbestimmungen an Stuhl routinemässig durchzuführen.

Das erfindungsgemässe Gefäss kann auch zum Probentransport der Stuhlprobe vom Patienten zum Labor eingesetzt werden ebenso wie zu der im Labor durchzuführenden Probenhomogenisierung im Gefässinnenraum, vorzugsweise durch Lösen des Zusatzdeckelteils, Zugabe von Homogenisierungsmittel und Homogenisierungselementen, vorzugsweise des Homogenisierungsstabs, Aufdrücken des Zusatzdeckelteils und anschliessendes Schütteln des Gefässes in einem Schüttelvibrator. Der eingefügte spiralige Homogenisierungsstab sorgt für einwandfreie Homogenisierung der relativ geringen Probenmenge, vor allem dann, wenn er aufgrund der konisch zulaufenden Gefässteilinnenfläche stets in die Probenausnehmung abgelenkt wird.

Schliesslich kann das erfindungsgemässe Gefäss auch zum Zentrifugieren durch Entfernen der Verbindungslaschen und Grifflaschen und anschliessendes Einsetzen des Gefässes in einer Zentrifuge verwendet werden. Nach der Probendosierung durch den Patienten können also sämtliche der eigentlichen, z.B. photometrischen Messung vorangehenden Bearbeitungsschritte am erfindungsgemässen Gefäss vorgenommen werden. Das Gefäss muss insgesamt nur einmal geöffnet werden, nämlich zur Entnahme eines Aliquot der homogenisierten und ggf. zentrifugierten Probenflüssigkeit. Das Laborpersonal hat bei sämtlichen Verfahrensstufen keinen direkten Kontakt mit der Probe. Nach der Entnahme eines Aliquot kann das Gefäss vernichtet werden.

Die Anwendung des erfindungsgemässen Gefässes ist nicht auf die Verarbeitung von Stuhlproben beschränkt, obgleich es hierfür besonders geeignet ist. Das Gefäss eignet sich generell für Dosierung und Homogenisierung (ggf. auch Zentrifugierung) pastöser Probenmaterialien (z.B. Pasten und Salben der Pharma- und Lebensmittelindustrie) zur Vorbereitung von Inhaltsstoffanalysen.

Die Erfindung wird im folgenden an mehreren Ausführungsbeispielen anhand der Zeichnung erläutert.

Es zeigt:

Fig. 1 einen Querschnitt durch eine erste Ausführungsform des erfindungsgemässen Gefässes;

Fig. 2 eine perspektivische Ansicht des Gefässes gemäss Fig. 1 in geöffnetem Zustand;

Fig. 3 einen Querschnitt durch eine zweite Ausführungsform;

Fig. 4 eine perspektivische Ansicht des Gefässes gemäss Fig. 3 in geöffnetem Zustand;

Fig. 5 einen Querschnitt durch eine dritte Ausführungsform;

Fig. 6 eine perspektivische Ansicht des Gefässes gemäss Fig. 5 in geöffnetem Zustand; und

Fig. 7 einen teilweisen Querschnitt durch eine weitere Ausführungsform.

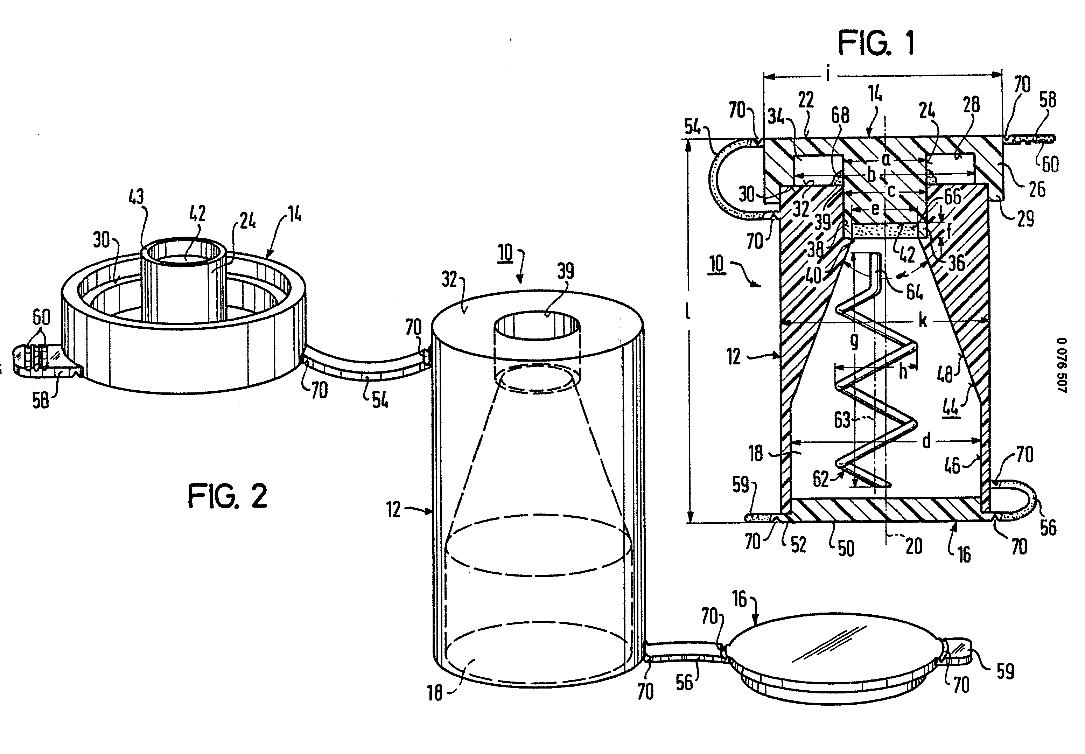

Die in den Fig. 1 und 2 dargestellte erste Ausführungsform des Gefässes ist mit 10 bezeichnet. Es besteht aus einem Gefässteil 12, einem Deckelteil 14 (oberer Deckel in den Fig. 1 und 2) und einem Zusatzdeckelteil 16 (unterer Deckel). Das Gefässteil 12 hat die Form eines Hohlzylinders (Röhrchenform); sein Innenraum wird als Gefässinnenraum 18 bezeichnet.

Der Aufbau des Deckelteils 14 geht aus den Fig. 1 und 2 hervor. Von einer zur Achse 20 senkrechten, ebenen Deckelplatte 22 stehen in den Fig. 1 und 2 nach unten (d. h. auf der Deckelteilinnenseite) ein zur Achse 20 zentrischer, zylindrischer Vorsprung 24 ab sowie ein zur Achse 20 symmetrischer hohlzylindrischer Rand 26, der bündig in den Aussenumfang der Deckelplatte 22 übergeht. Vorsprung 24 und Rand 26 legen zwischen sich in radialer Richtung eine in Fig. 1 nach unten offene Ringnut 28 fest. Der kleine Durchmesser der Ringnut 28, der dem Durchmesser des Vorsprungs 24 entspricht, ist in Fig. 1 mit a bezeichnet; dementsprechend der grosse Durchmesser der Ringnut mit b. Der Durchmesser b ist kleiner als der Aussendurchmesser k des zylindrischen Gefässteils 12. Um das Deckelteil 14 längs des Randes 26 dichtend auf das Gefässteil 12 aufstecken zu können, ist in den Rand 26 ein zylindrischer Einstich eingeformt, dessen Durchmesser dem Durchmesser k entspricht. Bei aufgesetztem Deckelteil 14 gemäss Fig. 1 liegt daher der Rand 26 mit einem innendurchmesservergrösserten Umfangsbund 29 abdichtend am Aussenumfang des Gefässteils 12 an. Bei vollständig aufgedrücktem Deckelteil 14 liegt darüber hinaus eine radiale Kreisringfläche 30 am in Fig. 1 oberen Ende des erwähnten Einstichs an der oberen Stirnfläche 32 des Gefässteils 12 abdichtend an. Man erhält auf diese Weise einen flüssigkeits- und gasdichten Abschluss eines bei aufgesetztem Deckelteil 14 gebildeten Ringraumes 34, der zum einen durch die erwähnte Ringnut 28 des Deckelteils 14 und zum anderen durch die Stirnfläche 32 des Gefässteils 12 begrenzt ist.

Wie Fig. 1 zeigt, füllt der zylindrische Vorsprung 24 mit seinem über die Ringnut 28 bzw. den Ringraum 34 in Fig. 1 nach unten vorstehenden Längenabschnitt 36 einen entsprechend dimensionierten zylindrischen Aufnahmeraum 38 des Gefässteils 12 mit einer axialen Raumlänge von ca. 5 bis 10 mm voll aus. An dem gefässinnenraumnächsten Ende des zylindrischen Aufnahmeraums 38 mit Innendurchmesser entsprechend dem Durchmesser c des Vorsprungs 36 ist ein radial nach innen vorstehender Innenumfangsbund 40 ausgebildet, an dessen in Fig. 1 nach oben gerichtete radiale Kreisringfläche der Vorsprung 24 mit seiner radialen Stirnfläche 43 bei vollständig aufgestecktem Deckelteil 14 anschlägt. Man erhält hierdurch eine doppelte Abdichtung des Gefässinnenraums 18 über das Deckelteil 14 nach aussen, nämlich über die Abdichtung zwischen dem Gefässteil 12 und dem Vorsprung 24 und über die Abdichtung zwischen dem Gefässteil 12 und dem Umfangsbund 29, und einen glatten Übergang zwischen dem Gefässinnenraum 18 und einer am freien Ende des Vorsprungs 24 (in Fig. 1 unten) stirnseitig eingeformten zylindrischen Probenausnehmung 42 zur Aufnahme von pastösem Probenmaterial.

Der Durchmesser e der Probenausnehmung 42 beträgt beispielsweise 5,6 mm; die Tiefe f der Ausnehmung beträgt beispielsweise 2 oder 4 mm. Dem entspricht im Falle einer Stuhlprobe ein Probengewicht von ca. 50 mg bzw. 100 mg. Das Volumen des Gefässinnenraums 18 beträgt dagegen ein Vielfaches des Volumens der Probenausnehmung 42, beispielsweise das 150-fache. Wie Fig. 1 zeigt, ist die den Gefässinnenraum 18 begrenzende Gefässteilinnenfläche 44 in einem in Fig. 1 unteren Abschnitt 46 zylindrisch ausgebildet und im nach oben sich anschliessenden Abschnitt 48 konisch ausgebildet. Der Durchmesser d des zylindrischen Abschnitts 46 ist mit 13 mm das 2- bis 3-fache des Durchmessers e der Probenausnehmung 42. Der konische Abschnitt 48 gleicht die Weite des Gefässinnenraums 18 an die der Probenausnehmung 42 an; der Durchmesser des Abschnitts 48 beträgt daher am unteren Konusende d und am oberen Konusende e. Der Konuswinkel α beträgt ca. 40°. Der konische Abschnitt 48 endet an seinem in Fig. 1 oberen Ende in dem erwähnten Innenumfangsbund 40.

Nach unten hin wird der Gefässinnenraum 18 durch das Zusatzdeckelteil 16 abgeschlossen, welches aus einer ebenen kreisförmigen Platte 50 besteht mit an den Durchmesser d des Gefässinnenraums 18 angepasstem Durchmesser, wobei auch eine an der zylindrischen Gefässinnenwand anliegende Wellendichtung des Zusatzdeckelteils in Frage kommt. Mit einem radial nach aussen abstehenden Rand 52 liegt das Zusatzdeckelteil 16 an der unteren Stirnfläche des Gefässteils 12 abdichtend an. Sowohl das Deckelteil 14 als auch das Zusatzdeckelteil 16 können jeweils mittels einer Verbindungslasche 54 bzw. 56 mit dem Gefässteil 12 einstückig verbunden sein. Das Deckelteil 14 ist ferner noch, der Verbindungslasche 54 gegenüberliegend, mit einer Grifflasche 58 versehen, die radial absteht und mit einer Riffelung 60 versehen sein kann. Die Verbindungslaschen 54 und 56 sorgen dafür, dass das Deckelteil 14 und Zusatzdeckelteil 16 unverlierbar am Gefässteil 12 angebracht sind. Die Grifflasche 58 erleichtert die Handhabung des Deckelteils 14 beim Dosieren der Probe. Eine entsprechende Grifflasche 59 am Zusatzdeckelteil 16 erleichtert dessen Lösen im Labor. Das aus Gefässteil 12, Deckelteil 14 und Zusatzdeckelteil 16 bestehende Gefäss kann einschliesslich der Laschen 54, 56, 58 und 59 als einstückiges Formteil, insbesondere Kunststoff-Spritzgussteil, ausgebildet sein.

In den Gefässinnenraum 18 ist ein in Fig. 1 schematisch dargestellter Homogenisierungsstab 62 einsetzbar. Dieser besteht aus einem spiralförmig gebogenen Draht- oder Kunststoffelement, dessen in Fig. 1 oberes Stabende 64 in Richtung der Spiralachse 63 umgebogen ist. Die axiale Länge g des Homogenisierungsstabes 62 ist etwas kleiner (z.B. 10 mm kleiner) als die axiale Gesamtlänge der Abschnitte 46 und 48 des Gefässinnenraums 18. Der Spiraldurchmesser h beträgt etwa 10 mm.

Das vorstehende Gefäss 10 eignet sich zur Probendosierung, Probenversand, Probenhomogenisierung und Probenzentrifugierung, wie im folgenden am Beispiel einer Stuhlprobe dargestellt werden wird, die einer Chymotrypsinuntersuchung unterzogen werden soll.

Vom Patienten wird mit einem Pappspatel eine Stuhlprobe in die Probenausnehmung 42 des geöffneten Deckelteils 14 eingefüllt und anschliessend mit flachliegendem Spatel entlang der die Probenausnehmung 42 umschliessenden radialen Kreisringfläche 43 (siehe Fig. 2) gestrichen, was einen sauberen Abschluss der Probenoberfläche mit dem Rand der Probenausnehmung 42 ergibt und damit ein wohl definiertes Probenvolumen. Die bei diesem Glattstreichen vom Spatel an dem Aussenumfang des zylindrischen Vorsprungs 24 im Bereich des freien Endes des Vorsprungs 24 ggf. abgelagerten überschüssigen Probenmengen müssen nicht manuell entfernt werden, sondern können dort verbleiben, da diese beim anschliessenden Schliessen des Deckelteils 14 automatisch aus dem Bereich des freien Endes des Vorsprungs 24 in den Ringraum 34 befördert werden und daher nicht in den Bereich des Gefässinnenraums 18 gelangen. Wird nämlich das Deckelteil 14 auf das Gefässteil 12 aufgesetzt, so gelangt als erstes der zylindrische Vorsprung 24 in den Aufnahmeraum 38, dessen Durchmesser dem des Vorsprungs 24 entspricht. Die der Stirnfläche 32 und dem Ringraum 38 gemeinsame, einen Abstreifkragen bildende Ringkante 39 streift daher das am Umfang des Vorsprungs 24 angelagerte überschüssige Probenmaterial ab, das sich im Ringraum 34 ansammelt. In Fig. 1 ist mit Punkten sowohl das in der Probenausnehmung 42 befindliche wohldosierte Probenmaterial 66 angedeutet, als auch das überschüssige Probenmaterial 68 im Ringraum 34. Bei geschlossenem Deckelteil 14 ist der Ringraum 34 über den Umfangsbund 29 nach aussen hin hermetisch abgedichtet, so dass es weder zu einer Geruchsbelästigung nach aussen hin kommt, noch gar zu einem Ausfliessen von Probenmaterial. Durch Ausbildung des Gefässes 10 aus mehr oder weniger undurchsichtigem Material kann auch eine optische Belästigung durch das unästhetische Probenmaterial vermieden werden. Bei der beschriebenen Probendosierung ist selbstverständlich das Zusatzdeckelteil 16 geschlossen.

Bei nicht ganz sachgemässer Handhabung des Pappspatels können nach dem Glattstreichen der Probe noch überschüssige Probenreste auf der die Probenausnehmung 42 umgreifenden radialen Kreisringfläche 43 des Vorsprungs 24 haften bleiben. Bei aufgedrücktem Deckelteil 14 werden diese überschüssigen Probenreste jedoch zwischen die Kreisringfläche 43 und die gegenüberliegende Kreisringfläche des Innenumfangsbundes 40 gepresst und auf diese Weise daran gehindert in den Gefässinnenraum einzudringen oder von Lösungsmittel im Gefässinnenraum aufgelöst zu werden.

Das in angegebener Weise mit einer Probe von beispielsweise 50 mg versehene, verschlossene Gefäss 10 kann unmittelbar zum Versand verwendet werden, ggf. eingesetzt in einem Transportbehälter.

Im Labor wird das Zusatzdeckelteil 16 geöffnet, der Homogenisierungsstab 62 und z.B. 5 ml Homogenisierungslösung zugefügt (Gefäss 10 um eine horizontale Achse um 180° gedreht gegenüber Fig. 1 und 2), darauf hin das Zusatzdeckelteil 16 verschlossen und anschliessend das ganze Gefäss 10 mit dem Deckelteil 14 nach unten in einen Schüttelvibrator eingesetzt. Hierbei wird das Gefässteil 12 in Richtung seiner Achse 20 hin- und hergeschüttelt, was aufgrund der Massenträgheit des Homogenisierungsstabs 62 eine Relativbewegung des Stabes 62 gegenüber dem Gefäss 10 im wesentlichen in Richtung der Achse 20 zur Folge hat. Aufgrund seiner Spiralstruktur führt der Stab 62 dabei Rotationsbewegungen in der einen und/oder anderen Richtung durch, was zur Folge hat, dass sich Lage und Orientierung des Stabes 62 ständig ändern und damit auch die Lage des oberen Stabendes 64, welches ausserhalb der Spiralachse liegen kann und zu dieser parallel ist. Der konische Abschnitt 48 lenkt den Stab 64 derart ab, dass das Stabende 64 stets in die Probenausnehmung 42 hineinstösst. Die Abmessungen des Stabes 64 (d.h. Stablänge g und Spiraldurchmesser h) sind derart auf die Abmessungen des Gefässinnenraums 18 angepasst, dass ausreichendes Spiel für den Stab 62 vorhanden ist, so dass das Stabende 64 während des Rüttelns praktisch an alle Stellen der Probenausnehmung gelangt, ohne dass es zu einem Umfallen des Stabes 62 kommt. Das Probenmaterial wird daher vom Stab 62 vollständig aus der Probenausnehmung 42 herausgeschlagen und durch die Stabwindungen zerkleinert.

Nach ca. einer halben Minute Rütteln ist eine ausreichend gute Homogenisierung erreicht. Anschliessend kann für manuelle Messungen das Zusatzdeckelteil 16 geöffnet werden und ein Aliquot (100 µl) der Suspension mit der Pipette für einen Labortest entnommen werden.

Man kann jedoch auch unmittelbar anschliessend an die Homogenisierung den Gefässinhalt zentrifugieren, z.B. für automatische Analysen. Hierzu werden im allgemeinen sämtliche Laschen 54, 56, 58 und 59 entfernt, zumindest jedoch die Laschen 54 und 58, um das Gefäss 10 in eine Zentrifugenöffnung einsetzen zu können. Der maximale Durchmesser i des Gefässes 10 ohne Laschen 54, 56 und 58 ist mit ca. 17 mm an den Durchmesser der Zentrifugenöffnung angepasst. Um das Abtrennen der Laschen 54, 56 und 58 zu erleichtern, sind in Fig. 1 angedeutete Sollbruchstellen 70 vorgesehen. Vorteilhafterweise wählt man die Gesamtlänge l des geschlossenen Gefässes 10 so, dass sie der Länge handelsüblicher, in die gebräuchlichen Zentrifugen einzusetzender Zentrifugengläser entspricht. Ggf. füllt man die Zentrifugenöffnung der verwendeten Zentrifuge, z.B. mit Gummistopfen, derart auf, dass das Zusatzdeckelteil 16 gerade noch über den Rand der Zentrifuge hinausragt. Nach der Zentrifugation wird das Zusatzdeckelteil geöffnet und wiederum eine Aliquot zur Bestimmung entnommen.

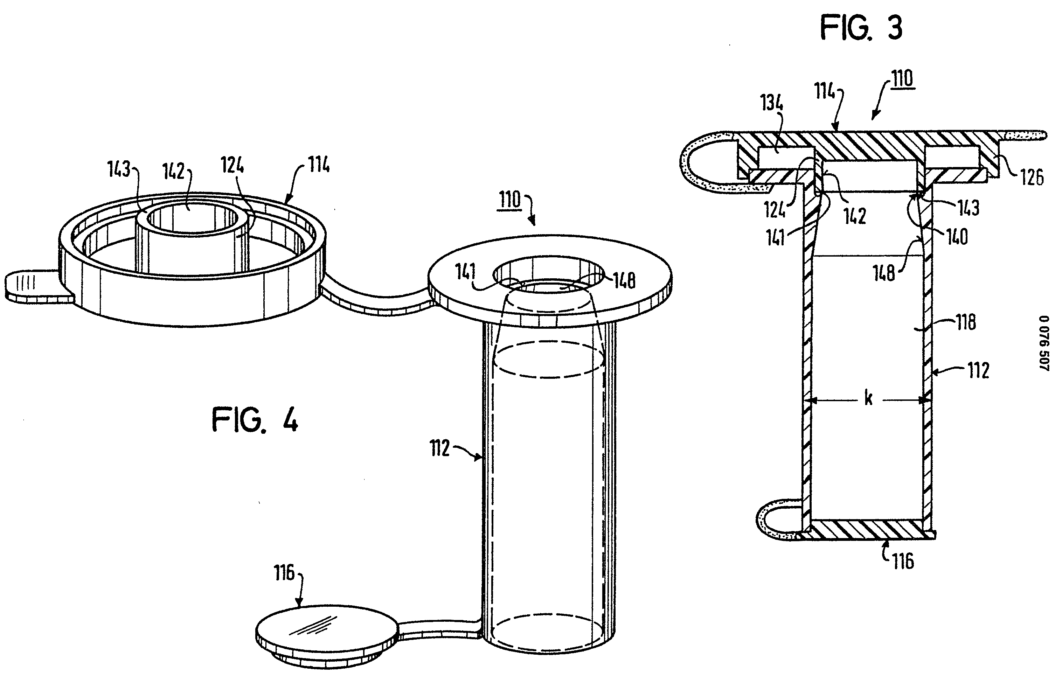

In den Fig. 3 und 4 ist eine zweite, mit 110 bezeichnete Ausführungsform des Gefässes dargestellt. Elemente des Gefässes 110, die denen des Gefässes 10 entsprechen, sind mit denselben Bezugsziffern versehen, jeweils vermehrt um die Zahl 100.

Das Gefäss 110 unterscheidet sich vom Gefäss 10 im wesentlichen lediglich darin, dass zur Materialeinsparung der Aussendurchmesser k des Gefässteils 112 reduziert worden ist bei entsprechender Vergrösserung der axialen Länge des Gefässteils 112, um unverändertes Volumen des Gefässinnenraums 118 sicherzustellen.

Das Deckelteil 114 weist gleichermassen einen Rand 126 zur Abdichtung des Ringraums 134 nach aussen hin auf sowie den Vorsprung 124 mit Probenausnehmung 142. Auch hier steht der Vorsprung 124 in axialer Richtung (in Fig. 3 nach unten) gegenüber dem Rand 126 vor, so dass beim Aufsetzen des Deckelteils 114 auf das Gefässteil 112 als erstes der Vorsprung 124 mit dem Gefässteil 112 in Berührung kommt.

Das Zusatzdeckelteil 116 dichtet den Gefässinnenraum 118 nach unten hin ab. Der Einsatz des kostengünstiger herstellbaren Gefässes 110 empfiehlt sich dann, wenn die Zentrifugierung entfallen kann.

Zu erwähnen ist noch, dass der radial nach innen vorstehende Innenumfangsbund 140, der das obere Ende des konischen Abschnittes 148 bildet, an seiner oberen Seite hinterschnitten ist. Bei aufgedrücktem Deckelteil 114 liegt der Innenumfangsbund 140 mit seiner Innenkante 141 am Vorsprung 124 nach Art einer Lippendichtung an und zwar im Bereich des inneren Randes der Kreisringfläche 143 (siehe Fig. 4). Überschüssige Probenreste, die bei nicht ganz sachgemässer Handhabung des Pappspatels an der Kreisringfläche 143 haften bleiben, werden daher bei aufgedrücktem Deckelteil 114 zuverlässig in dem durch die Hinterschneidung und die Kreisringfläche 143 gebildeten, nach innen hin abgedichteten Ringraum 143 festgehalten.

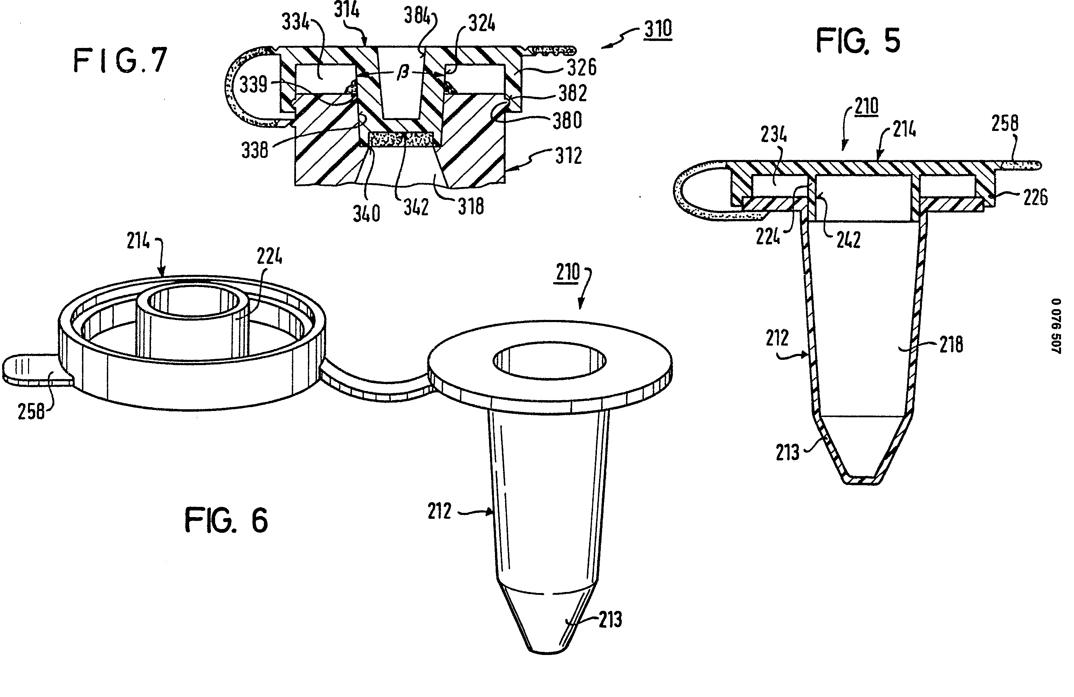

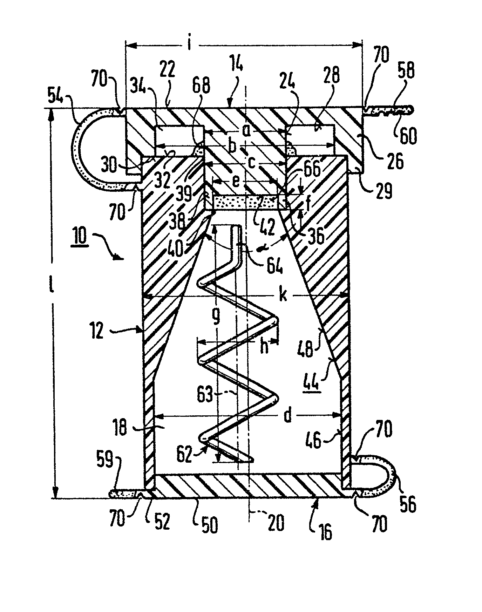

In den Fig. 5 und 6 ist schliesslich noch eine mit 210 bezeichnete dritte Ausführungsform des Gefässes dargestellt, die gegenüber den Gefässen 10 und 110 noch weiter vereinfacht ist. Elemente des Gefässes 210, die Elementen des Gefässes 10 gemäss Fig. 1 und 2 entsprechen, sind mit denselben Bezugsziffern, jeweils vermehrt um die Zahl 200, versehen.

Das Gefäss 210 unterscheidet sich von den Gefässen 10 bzw. 110 darin, dass das Zusatzdeckelteil 16 bzw. 116 entfällt. Statt dessen ist das Gefässteil 212 nach unten hin durch eine entsprechende Gefässabrundung 213 geschlossen. Das Deckelteil 214 hat dagegen den gleichen Aufbau wie die Deckelteile 14 bzw. 114 mit Rand 226 und Vorsprung 224. Die Probenausnehmung 242 ist jedoch in axialer Richtung tiefer ausgebildet zur Aufnahme grösserer Probenvolumina. In dieser besonders kostengünstig herstellbaren Ausführungsform entfällt der konusartige Abschnitt 48 gemäss Fig. 1 unterhalb des Randes der Probenausnehmung. Die Handhabung des Gefässes 210 bei der Probendosierung ist die gleiche wie bei den Gefässen 10 und 110. Zur anschliessenden Homogenisierung bieten sich folgende Varianten an:

a) Das Gefäss 210 wird im Labor anhand der Grifflasche 258 geöffnet und anschliessend Homogenisierungsmittel und einige Homogenisierungskugeln, -stäbe oder -spiralen etc. eingefüllt. Nach erneutem Verschliessen des Gefässes 210 wird das Gefäss mit dem Deckelteil 214 nach unten auf den Schüttelvibrator gesetzt und die Probe unter der Wirkung der Kugeln aus der Probenausnehmung 242 gelöst und homogenisiert.

b) Das Gefäss 10 kann jedoch auch bereits vom Hersteller mit Homogenisierungsmittel und Homogenisierungskugeln, -stäben oder -spiralen etc. gefüllt sein. Der Patient hat dann unmittelbar vor dem Aufdrücken des mit Probe versehenen Deckelteils 214 eine Folie abzuziehen, die den mit Homogenisierungsmittel und Homogenisierungskugeln versehenen Gefässinnenraum 218 bis dahin abschliesst.

c) Man kann jedoch auch die Folie gemäss Variante b) derart gestalten, dass sie vom Rand der Probenausnehmung 242 beim Aufdrücken des Deckelteils 214 auf das Gefässteil 212 zerrissen und abgelöst wird.

Nach der Homogenisierung kann zur Probenentnahme das Gefäss 210 kurz geöffnet und ein Aliquot entnommen werden. Alternativ hierzu kann man jedoch auch das geschlossene Gefäss, insbesondere das Deckelteil 214, mit einer Kanüle durchstechen und ein Aliquot entnehmen. Im Falle der Varianten b) und c) erübrigt sich bei einer Probenentnahme mittels Kanüle jegliches Öffnen des Gefässes 210 im Labor.

In Fig. 7 ist eine weitere mit 310 bezeichnete Ausführungsform des Gefässes dargestellt, wobei Elemente dieses Gefässes, die denen des Gefässes 10 gemäss Fig. 1 und 2 entsprechen, mit denselben Bezugsziffern, jeweils vermehrt um die Zahl 300, versehen sind. Das Gefäss 310 unterscheidet sich vom Gefäss 10 zum einen darin, dass der Vorsprung 342 nicht zylindrisch sondern in Richtung zur Probenausnehmung 342 leicht konisch zuläuft (Konuswinkel β ca. 5°). Die Aufnahme 338 ist nach wie vor zylindrisch, wobei der Zylinderdurchmesser derart festgelegt ist, dass zu Beginn der Einsteckbewegung des Vorsprungs 342 in den Aufnahmeraum 338 geringes Spiel zwischen der Ringkante 339 am oberen Ende des Aufnahmeraums 338 und dem in Fig. 7 unteren Ende des Vorsprungs 324 besteht. Der Durchmesser des Aufnahmeraums 339 ist jedoch geringer als der Durchmesser des unverformten konischen Vorsprungs 324 in Höhe der Ringkante 339 bei als vollständig in die Aufnahme 339 eingesteckt gedachtem Vorsprung 324. Aufgrund dieser Durchmesserdifferenz kommt es bei vollständig eingestecktem Vorsprung 324 zu einer gegenseitigen Verformung von Gehäuseteil 312 und Deckelteil 314 bzw. Vorsprung 324 im Bereich der Ringkante 339. Der entsprechend erhöhte Reibungskraftschluss mit Selbsthemmung zwischen den beiden Teilen verhindert ein unbeabsichtigtes Lösen beidet Teile voneinander. Ferner erhält man einen absoluten dichten Abschluss zwischen Gehäuseinnenraum 318 und Ringraum 334 zwischen Gefässteil 312 und Deckelteil 314.

Das Gefäss 310 unterscheidet sich vom Gefäss 10 gemäss Fig. 1 und 2 ferner dadurch, dass der axial in gleicher Richtung wie der Vorsprung 324 vorstehende Umfangsrand 326 am Aussenumfang des Gefässteils 314 über eine Schnappverbindung festgelegt ist. Diese Schnappverbindung besteht aus einem wulstförmigen Umfangsvorsprung 380, z.B. am Aussenumfang des Gehäuseteils 312, und einer komplementär ausgebildeten Umfangsausnehmung 382 am Innenumfang des Randes 326. Bedarfsweise können auch mehrere derartige Umfangsvorsprünge bzw. -Ausnehmungen vorgesehen sein. Diese Schnappverbindung (mit Vergrösserung der unter Vorspannung aneinander anliegenden Flächen von Rand 326 und Gefässteil 312) bildet eine hermetische Abdichtung des Ringraums 334 nach aussen. Zudem wird hierdurch der Halt des geschlossenen Deckelteils 314 am Gefässteil 312 verbessert und ein unbeabsichtigtes Öffnen zumindest stark erschwert.

Gemäss Fig. 7 ist der Rand 326 im Gegensatz zu den vorstehend beschriebenen Ausführungsformen nicht abgestuft, was verringerten Materialeinsatz zur Folge hat, ebenso wie die Ausbildung des Vorsprungs 324 mit einer zur Deckelaussenseite hin offenen Aussparung 384. Im Bedarfsfalle kann natürlich der Rand 326 entsprechend den Fig. 1 bis 6 auch abgestuft ausgebildet sein, was das korrekte Zusammenfügen von Umfangsvorsprung 380 und Umfangsausnehmung 382 erleichtert.

Die erfindungsgemässe Vorrichtung hat sich als besonders zweckmässig für die Durchführung des in der Europäischen Patentanmeldung EP-A-0 076 506 unter der Bezeichnung «Verfahren zur Bestimmung der Aktivität von Chymotrypsin oder Trypsin im Stuhl» der Anmelderin beschriebenen Verfahrens erwiesen. Bei diesen Verfahren werden relativ geringe, genau dosierte Probenmengen in Gegenwart eines oberflächenaktiven Mittels suspendiert und anschliessend die Geschwindigkeit der Spaltung eines geeigneten Substrats durch die Stuhlsuspension gemessen.

Die Messung der Geschwindigkeit der Spaltung des Substrats kann nach einer der aus dem Stand der Technik dafür bekannten Methoden erfolgen, wie z.B. durch Titration der freigesetzten Aminosäure mittels Lauge (pH-stat-Verfahren). Dabei hat es sich gezeigt, dass bei der erfindungsgemässen Durchführung des Verfahrens in Gegenwart eines oberflächenaktiven Mittels im allgemeinen mehr als 90% der Enzymaktivität solubilisiert sind, die Reaktionsgeschwindigkeit der Substratspaltung beträchtlich erhöht und der scheinbare Km-Wert des Substrats herabgesetzt ist (Aktivierungsfaktor ca. 2 bis 10), wodurch die bisher auftretenden Probleme (insbesondere Bindung des Enzyms an die Stuhlpartikel und überraschend grosse Km-Werte für unbehandelte Stuhlproben im Vergleich zu kristallinem Enzym bei bestimmten Substraten) überwunden werden können. Insbesondere ist es dadurch möglich, die Geschwindigkeit der Spaltung des Substrats auch auf photometrischem Wege rasch, einfach, genau und mit geringem Substrateinsatz zu messen. Die mit dem erfindungsgemässen Verfahren in Gegenwart eines oberflächenaktiven Mittels erhaltenen Ergebnisse sind insbesondere auch deshalb überraschend, weil bei reinem α-Chymotrypsin aus Rinderpankreas praktisch kein Unterschied bei der Durchführung der Bestimmungsmethode in oder ohne Gegenwart eines oberflächenaktiven Mittels festgestellt wurde.

Als oberflächenaktives Mittel kann im Prinzip jedes geeignete Tensid eingesetzt werden, wie anionische oder ampholytische Tenside, und vorzugsweise nichtionogene Tenside und insbesondere kationische Tenside.

Anionische Tenside sind zum Beispiel Alkansulfonate, Olefinsulfonate, z.B. Cumensulfonat, Estersulfonate, Alkylarylsulfonate, insbesondere Alkylbenzolsulfonate vom Typ des Dodecylbenzolsulfonats und Alkylnaphthalinsulfonate, Alkylsulfate, wie z.B. Natrium-laurylsulfat, Äthersulfate oder Fettalkoholsulfate, Salze von Fettsäuren und Cholsäuren; ampholytische Tenside sind solche mit anionenaktiven und kationenaktiven hydrophilen Gruppen, wie z.B. Glycerinderivate mit Betain-Struktur, Sulfobetaine und Lecithine; nichtionogene Tenside sind z.B. Polyäther, insbesondere Alkylphenolpolyglykoläther und andere Produkte der Ätholxylierung von Fettsäuren, Fettsäureamiden, Fettaminen und Fettalkoholen, z.B. äthoxylierter Laurylalkohol, Polymere aus Propylen und Äthylenoxid, Polyoxyäthylen-alkyläther oder -nonylphenyläther, Polyoxyäthylensorbitan-monooleat oder Laureat, Additionsprodukte aus Propylenoxid/Äthylendiamin/Äthylenoxid, Aminoxide und Fettsäureester von Polyalkoholen, Talg-Alkohol-Polyglykoläther; kationische Tenside sind z.B. geradkettige und cyclische Ammoniumverbindungen, z.B. N-Cetyl-N-äthylmorpholinmetosulfat, Benzalkoniumchloride und andere quartäre Ammoniumsalze, Aminsalze, Pyridiniumsalze oder quaternäre Fettamin-polyglykoläther.

Die Wahl des am besten geeigneten Tensids ist auch von den übrigen Reaktionsbedingungen abhängig, insbesondere von der Art des Enzyms und Substrats, von der Art und Konzentration der Salze, aber auch vom pH-Wert des Mediums.

Von den für das erfindungsgemässe Verfahren in erster Linie geeigneten kationischen Detergentien zeigen den stärksten Aktivierungseffekt quartäre Ammoniumverbindungen, vorzugsweise der Formel $R_1R_2N^{\oplus}(CH_3)_2$, worin $R_1$ vorzugsweise einen Alkylrest mit 8 bis 14 Kohlenstoffatomen, und insbesondere Lauryl und Cetyl bedeutet, und $R_2$ vorzugsweise einen Niederalkylrest mit 1 bis 5 Kohlenstoffatomen oder einen Aralkylrest, oder auch einen Hydroxyalkylrest, und insbesondere Methyl- oder Benzyl, und Alkylpyridiniumsalze mit vorzugsweise 12 bis 18 Kohlenstoffatomen im Alkylrest, wie z.B. Laurylpyridiniumchlorid, Laurylpyridiniumdisulfat, und insbesondere das Hexadecylpyridiniumchlorid. Ein für das erfindungsgemässe Verfahren besonders gut geeignetes Tensid ist das Lauryl-trimethylammoniumchlorid.

Die Konzentration des oberflächenaktiven Mittels in der zur Suspension des Stuhls verwendeten Homogenisierlösung beträgt im allgemeinen ca. 0,02 bis ca. 10 Gew.-%, vorzugsweise 0,5 bis 5 Gew.-%. Die Konzentration des oberflächenaktiven Mittels in der Messlösung (Stuhlsuspension und Substratlösung) sollte zweckmässigerweise ca. 0,05 bis 0,5, und vorzugsweise 0,1 bis 0,3 Gew.-% betragen. Die Konzentration der Stuhlprobe in der Suspension beträgt z.B. bei Verwendung des Substrats Succ-Ala-Ala-Pro-Phe-pNA 0,2 bis 0,5%.

Vorzugsweise enthält die zur Suspension des Stuhls verwendete Homogenisierlösung neben dem oberflächenaktiven Mittel noch ein oder mehrere wasserlösliche Salze, wie z.B. Alkali- und/oder Erdalkalichloride oder Sulfat. Als besonders zweckmässig hat sich ein Gehalt an NaCl von 100 bis 1000 mMol/l oder ein Gehalt an $CaCl_2$ von 20 bis 500 mMol/l oder eine Kombination beider Salze in den genannten Konzentrationsbereichen erwiesen. Es eignen sich aber auch organische Salze, z.B. Acetate oder Citrate, sowie Salze anderer Kationen. Die Salze sollten zweckmässig in einer Konzentration vorliegen, die einer Ionenstärke von 20 bis 1000 mVal/l entspricht.

Dabei wurde überraschend festgestellt, dass durch eine ein oberflächenaktives Mittel und Salze (Ionenstärke) enthaltende Homogenisierlösung eine überadditive Steigerung der Enzymaktivität (Erhöhung der Reaktionsgeschwindigkeit) eintritt, d.h. die Steigerung ist grösser als die Summe der in Gegenwart von oberflächenaktivem Mittel oder in Gegenwart von Salzen erhaltenen Werte. Desweiteren bringt erst die Kombination von Salz und Detergens – im Gegensatz zu den Einzelkomponenten – einen konstant hohen und repräsentativen Teil des partikelgebundenen Enzyms in Lösung.

Als Substrat kann im allgemeinen jedes aus dem Stand der Technik für die Bestimmung der Aktivität von Chymotrypsin oder Trypsin im Stuhl nach den bisherigen Methoden als geeignet bekanntes Substrat verwendet werden (vgl. z.B. Grossman, Proc. Soc. Biol. Med. 110 (1962), 41; Del Mar et al., Anal. Biochem. 99 (1979) 316; Nakajama et al., J. Biol. Chem. 254 (10), 1979, 4027–4032). Für die Chymotrypsin-Bestimmung als besonders geeignet, vor allem im Hinblick auf Wasserlöslichkeit, Stabilität, Km-Wert und Geschwindigkeitskonstante, erwiesen sich das Ala-Ala-Phe-pNA und insbesondere das Succ-Ala-Ala-Pro-Phe-pNA und MeO-Succ-Arg-Pro-Tyr-pNA; sie eignen sich insbesondere sehr gut für eine photometrische Bestimmung. Ein für die Bestimmung der Trypsin-Aktivität gut geeignetes Substrat ist z.B. Chromozym TRY (Carbobenzoxy-valyl-glycylarginin-p-nitroanilid-acetat).

Zur Herstellung der Reagenslösung wird das Substrat in Wasser oder einem Gemisch aus Wasser und einem organischen Lösungsmittel gelöst. Das organische Lösungsmittel dient dabei als Lösungsvermittler für das Substrat, und ist z.B. Acetonitril, Dimethylsulfoxid, Aceton oder Methanol. Die Reagenslösung enthält vorzugsweise auch noch die gleichen Salze wie sie für die Homogenisierlösung verwendet werden; die Konzentration dieser Salze in der Reagenslösung ist im allgemeinen niedriger als die Konzentration in der Homogenisierlösung; die gesamte Salzkonzentration beträgt vorzugsweise ca. 50 bis 500 mMol/l, z.B. 250 mMol/l Natriumchlorid und 20 mMol/l Calciumchlorid.

Zur Durchführung der Messung wird eine bestimmte Menge einer Suspension der Stuhlprobe in der Homogenisierlösung oder des durch Zentrifugation bis zur Klärung erhaltenen Zentrifugationsüberstands zu einer bestimmten Menge der Reagenslösung zugegeben (z.B. 100 µl Probensuspension zu 2 ml Reagenslösung) und die Geschwindigkeit der Spaltung z.B. titrimetisch oder photometrisch bestimmt. Die Menge an Probensuspension (Probenverdünnung) richtet sich dabei zur Erzielung gut messbarer Werte, z.B. gut messbarer Extinktionsanstiege/Minute, vor allem nach der zu erwartenden Enzymaktivität. Aufgrund der Empfindlichkeitssteigerung durch Einsatz des Detergens kann die Probenmenge so klein gewählt werden, dass die Eigenabsorption der suspendierten Festkörper gering ist und somit eine photometrische Messung mit der Suspension erst ermöglicht wird. Der pH-Wert der Homogenisierlösung ist nicht besonders kritisch; er liegt im allgemeinen zwischen 3 und 10, und insbesondere zwischen 6 und 8. Der pH-Wert der Mischung aus Probensuspension und Reagenslösung liegt im allgemeinen zwischen 8 und 10, und vorzugsweise bei pH = 9; diese Werte werden vorzugsweise über den pH-Wert der Reagenslösung eingestellt. Es kann auch vorteilhaft sein, den Lösungen zur Einstellung des pH-Werts ein geeignetes Puffergemisch zuzugeben, wie z.B. Tris-Puffer, Glycin-Puffer oder Glycylglycin-Puffer. Die Pufferkonzentration liegt im allgemeinen zwischen 10 und 1000 mMol/l, insbesondere bei 50 bis 200 mMol/l.

Die Messung kann manuell oder mit einem automatischen Analysengerät durchgeführt werden.

Für die photometrische Aktivitätsbestimmung, und insbesondere zur automatischen photometrischen Messung (Bestimmung der Extinktion) ist es zweckmässig, die Probensuspension vor der Messung bis zur Klärung zu zentrifugieren.

**Patentansprüche**

1. Gefäss zur Handhabung von pastösem Probenmaterial mit einem Gefässteil (12, 112, 212, 312) und einem auf das Gefässteil aufsteckbaren, einen Gefässinnenraum (18, 118, 218, 318) abschliessenden Deckelteil (14, 114, 214, 314), wobei an der dem Gefässinnenraum (18, 118, 218, 318) zugewandten Deckelteilinnenseite ein Vorsprung (24, 124, 224, 324) im wesentlichen senkrecht absteht, an dessen freiem Ende stirnseitig eine zum Gefässinnenraum (18, 118, 218, 318) offene Probenausnehmung (42, 142, 242, 342) zur Aufnahme des Probenmaterials (66) ausgebildet ist, deren Volumen dem gewünschten Probenvolumen entspricht, dadurch gekennzeichnet, dass das Gefässteil (12, 112, 212, 312) im Bereich seiner Öffnung für das Deckelteil (14, 114, 214, 314) mit einem an dem Querschnitt des Vorsprungs (24, 124, 224, 324) angepassten Abstreifkragen (39, 339) für am Umfang des Vorsprungs (24, 124, 224, 324) angelagertes Probenmaterial (68) ausgebildet ist, und dass auf der vom Gefässinnenraum (18, 118, 218, 318) abgelegenen Seite des Abstreifkragens (39) im Deckelteil (14, 114, 214, 314) und/oder im Gefässteil ein nach aussen hin abschliessender Ringraum (34, 134, 234, 334) zur Aufnahme abgestreiften Probenmaterials (68) ausgebildet ist.

2. Gefäss nach Anspruch 1, dadurch gekennzeichnet, dass das Deckelteil (14, 114, 214, 314) und Gefässteil (12, 112, 212, 312) mit einer den Ringraum (34, 134, 234, 334) nach aussen hin abdichtenden Umfangsdichtung ausgebildet sind, vorzugsweise in Form eines an der Deckelteilinnenseite vorstehenden Umfangsbundes (29, 329), der mit einer Innenumfangsfläche an einer Aussenumfangsfläche einer Umfangsschulter des Gefässteils (12, 112, 212, 312) abdichtend anliegt.

3. Gefäss nach Anspruch 2, dadurch gekennzeichnet, dass der Vorsprung (24, 124, 224, 324) über den Umfangsbund (29, 329) vorsteht, vorzugsweise 1 bis 5 mm, am besten etwa 2 mm.

4. Gefäss nach einem der vorgehenden Ansprüche, dadurch gekennzeichnet, dass das Gefässteil (12, 112) mit einer mit dem Gefässinnenraum (18, 118) verbundenen weiteren Öffnung versehen ist, die mit einem wieder entfernbaren Zusatzdeckelteil (16, 116) verschlossen ist.

5. Gefäss nach Anspruch 4, dadurch gekennzeichnet, dass das Gefässteil (12, 112) röhrchenförmig ist und an einem seiner Rohrenden mit dem Deckelteil (14, 114) und am anderen Rohrende mit dem Zusatzdeckelteil (16, 116) verschlossen ist.

6. Gefäss nach einem der vorhergehenden Ansprüche, dadurch gekennzeichnet, dass das Deckelteil (14, 114, 214, 314) und/oder das Zusatzdeckelteil (16, 116) je über eine Verbindungslasche (54; 56) vorzugsweise einstückig mit dem Gefässteil (12, 112, 312) verbunden sind.

7. Gefäss nach einem der vorhergehenden Ansprüche, dadurch gekennzeichnet, dass das Deckelteil (14, 114, 214, 314) und/oder das Zusatzdeckelteil (16, 116) mit einer Grifflasche (58, 59, 258) vorzugsweise einstückig verbunden sind.

8. Gefäss nach einem der vorhergehenden Ansprüche, dadurch gekennzeichnet, dass das Gefässteil (12) und das Deckel- und/oder das Zusatzdeckelteil (14, 16) einen Aussendurchmesser (i) von ca. 17 mm aufweisen, und dass ggf. die Verbindungs- und Grifflaschen (54, 56, 58, 59) vorzugsweise über Sollbruchstellen (70) leicht abtrennbar sind.

9. Gefäss nach einem der vorhergehenden Ansprüche, dadurch gekennzeichnet, dass die den Gefässinnenraum (18, 118) begrenzende Gefässteilinnenfläche (44) zur Probenausnehmung (42, 142) im Deckelteil (14, 114) hin vorzugsweise konisch zuläuft.

10. Gefäss nach einem der vorhergehenden Ansprüche, dadurch gekennzeichnet, dass das Gefässteil (12; 112) mit einem vorzugsweise hinterschnittenen Innenumfangsbund (40; 140, 340) an der Stirnseite (43; 143) des Vorsprungs (24; 124; 324) im Bereich des Randes der Probenausnehmung (42, 142, 342) anliegt.

11. Gefäss nach einem der vorhergehenden Ansprüche, gekennzeichnet durch einen in den Gefässinnenraum (18, 118) einsetzbaren, spiralig ausgebildeten Homogenisierungsstab (62) dessen Spiraldurchmesser (h) an dem Gefässinnenraumdurchmesser (d) angepasst ist.

12. Gefäss nach Anspruch 11, dadurch gekennzeichnet, dass das der Probenausnehmung (42, 142) nahe Stabende (64) des Homogenisierungsstabs (62) sich im wesentlichen in Richtung der Spiralachse (63) erstreckt, vorzugsweise mit Abstand zur Spiralachse (63).

13. Gefäss nach einem der vorhergehenden Ansprüche, dadurch gekennzeichnet, dass der Vorsprung (324) in Richtung zur Probenausnehmung (342) leicht konisch zuläuft.

14. Gefäss nach einem der vorhergehenden Ansprüche, gekennzeichnet durch einen Aufnahmeraum (38, 338) für den Vorsprung (24, 324) im Gefässteil (12, 312), an dessen Vorsprung-Einsteckende der Abstreifkragen (39, 339) angeordnet ist und an dessen gegenüberliegendem, in den Gefässinnenraum (18, 318) übergehenden Ende ggf. der hinterschnittene Innenumfangsbund (40, 340) angeordnet ist.

15. Gefäss nach Anspruch 14, dadurch gekennzeichnet, dass der Aufnahmeraum (38, 338) zylindrisch oder auf den Gefässinnenraum leicht konisch zulaufend ausgebildet ist.

16. Gefäss nach einem der Ansprüche 2 bis 15, dadurch gekennzeichnet, dass der Umfangsbund (326) an der Umfangsschulter des Gefässteils (312) mittels einer Schnappverbindung festlegbar ist, vorzugsweise in Form wenigstens einer an einem der beiden Teile vorgesehenen Umfangserhebung (380), welche in eine am jeweils anderen Teil vorgesehene Umfangsvertiefung (382) eingreift.

17. Verwendung des Gefässes nach einem der vorhergehenden Ansprüche zur Stuhlprobendosierung von Mengen kleiner als 1 g Stuhl, vorzugsweise zwischen 20 und 200 mg Stuhl, insbesondere zur Enzymbestimmung, vorzugsweise Chymotrypsin- oder Trypsinbestimmung im Stuhl.

18. Verwendung des Gefässes nach Anspruch 17 zum Probentransport, insbesondere Probenversand.

19. Verwendung des Gefässes nach einem der Ansprüche 17 und 18 zur Probenhomogenisierung im Gefässinnenraum, vorzugsweise durch Lösen des Zusatzdeckelteils, Zugabe von Homogenisierungsmittel und Homogenisierungselementen, vorzugsweise des Homogenisierungsstabs, Aufdrücken des Zusatzdeckelteils und anschliessendes Schütteln des Gefässes in einem Schüttelvibrator.

20. Verwendung des Gefässes nach einem der Ansprüche 17 bis 19 zur Zentrifugierung durch Entfernen der Verbindungslaschen und Grifflaschen und anschliessenden Einsetzen des Gefässes in einer Zentrifuge.

**Claims**

1. Receptacle for the handling of pasty sample material with a receptacle part (12, 112, 212, 312) and a cover part (14, 114, 214, 314) mountable on the receptacle part and closing a receptacle inner chamber (18, 118, 218, 318), whereby, on the side of the cover part facing the receptacle inner chamber (18, 118, 218, 318), a projection (24, 124, 224, 324) projects essentially vertically, on the free end of which is formed on the face a sample recess (42, 142, 242, 342) open towards the receptacle inner chamber (18, 118, 218, 318) for the reception of the sample material (66), the volume of which corresponds to the desired sample volume, characterised in that the receptacle part (12, 112, 212, 312), in the region of its opening for the cover part (14, 114, 214, 314) is formed with a wipe-off collar (39, 339), adapted to the cross-section of the projection (24, 124, 224, 324), for sample material (68) deposited on the circumference of the projection (24, 124, 224, 324) and that on the side of the wipe-off collar (39) remote from the receptacle inner chamber (18, 118, 218, 318) there is formed, in the cover part (14, 114, 214, 314) and/or in the receptacle part, an outwardly closing annular chamber (34, 134, 234, 334) for the reception of wiped-off sample material (68).

2. Receptacle according to claim 1, characterised in that the cover part (14, 114, 214, 314) and the receptacle part (12, 112, 212, 312) are formed with a circular seal outwardly sealing the annular chamber (34, 134, 234, 334), preferably in the form of a circumferential flange (29, 329) projecting on the cover part inner side, which lies sealingly with an inner circumferential surface on an outer circumferential surface of the receptacle part (12, 112, 212, 312).

3. Receptacle according to claim 2, characterised in that the projection (24, 124, 224, 324) projects over the circumferential flange (29, 329), preferably 1 to 5 mm., best about 2 mm.

4. Receptacle according to one of the preceding claims, characterised in that the receptacle part (12, 112) is provided with a further opening, connected with the receptacle inner chamber (18, 118), which is closed with a removable additional cover part (16, 116).

5. Receptacle according to claim 4, characterised in that the receptacle part (12, 112) is tubular shaped and is closed at one of its tube ends with the cover part (14, 114) and at the other tube end with an additional cover part (16, 116).

6. Receptacle according to one of the preceding claims, characterised in that the cover part (14, 114, 214, 314) and/or the additional cover part (16, 116) are each connected via a connecting piece (54; 56), preferably in one piece, with the receptacle part (12, 112, 312).

7. Receptacle according to one of the preceding claims, characterised in that the cover part (14, 114, 214, 314) and/or the additional cover part (16, 116) are connected with a gripping piece (58, 59, 258).

8. Receptacle according to one of the preceding claims, characterised in that the receptacle part (12) and the cover and/or the additional cover part (14, 16) have an outer diameter (i) of about 17 mm. and that the connecting and gripping pieces (54, 56, 58, 59) can possibly be easily separated, preferably via prearranged breaking points (70).

9. Receptacle according to one of the preceding claims, characterised in that the receptacle part inner surface (44) bounding the receptacle inner part (18, 118) runs towards the sample recess (42, 142) in the cover part (14, 114) preferably conically.

10. Receptacle according to one of the preceding claims, characterised in that receptacle part (12, 112) with a preferably undercut inner circumferential flange (40; 140, 340) abuts on the end face (43; 143) of the projection (24; 124; 324) in the region of the edge of the sample recess (42, 142, 342).

11. Receptacle according to one of the preceding claims, characterised by a homogenising rod (62), insertable into the receptacle inner chamber (18, 118) and formed spirally, the spiral diameter (h) of which is adapted to the receptacle inner chamber diameter (d).

12. Receptacle according to claim 11, characterised in that the sample recess (42, 142) extends near the rod end (64) of the homogenising rod (62) essentially in the direction of the spiral axis (63), preferably distanced from the spiral axis (63).

13. Receptacle according to one of the preceding claims, characterised in that the projection (324) runs slightly conically in the direction of the sample recess (342).

14. Receptacle according to one of the preceding claims, characterised by a reception chamber (38, 338) for the projection (24, 324) in the receptacle part (12, 312), on the projection insertion end of which is arranged the wipe-off collar (39, 339) and on the opposite end of which, passing over into the receptacle inner chamber (18, 318), is possibly provided the undercut inner circumferential flange (40, 340).

15. Receptacle according to claim 14, characterised in that the reception chamber (38, 338) is constructed cylindrically or running slightly conically towards the receptacle inner chamber.

16. Receptacle according to one of claims 2 to 15, characterised in that circumferential flange (326) can be securely laid on the circumferential shoulder of the receptacle part (312) by means of a snap connection, preferably in the form of at least one circumferential elevation (380) provided on one of the two parts which engages in a circumferential recess (382) provided on the other part.

17. Use of the receptacle according to one of the preceding claims for faecal sample measuring of amounts of less than 1 g. of faeces, preferably between 20 and 200 mg. of faeces, especially for enzyme determination, preferably chymotrypsin or trypsin determination in faeces.

18. Use of the receptacle according to claim 17 for sample transport, especially sample despatch.

19. Use of the receptacle according to one of claims 17 and 18 for the sample homogenisation in the receptacle inner chamber, preferably by loosening of the additional cover part, addition of homogenising agent and homogenising elements, preferably of the homogenising rod, pressing on of the additional cover part and subsequent shaking of the receptacle in a shaking vibrator.

20. Use of the receptacle according to one of claims 17 to 19 for centrifuging after removal of the connecting pieces and gripping pieces and subsequent insertion of the receptacle into a centrifuge.

**Revendications**

1. Récipient permettant la manipulation d'un échantillon constitué par une matière pâteuse, comprenant une section de récipient (12, 112, 212, 312) et un élément formant couvercle (14, 114, 214, 314) pouvant être placé sur la section de récipient, fermant une cavité intérieure (18, 118, 218, 318) du récipient, la face intérieure de la partie formant couvercle située du côté de la cavité intérieure (18, 118, 218, 318) du récipient comportant une saillie (24, 124, 224, 324) qui en part sensiblement perpendiculairement, à l'extrémité libre de laquelle est formé frontalement un évidement de réception d'échantillon (42, 142, 242, 342) ouvert vers la cavité intérieure (18, 118, 218, 318) du récipient, devant loger la matière constituant l'échantillon (66), dont le volume correspond au volume d'échantillon souhaité, caractérisé en ce que la section de récipient (12, 112, 212, 312) comporte, dans la zone de son ouverture pour l'élément formant couvercle (14, 114, 214, 314) une collerette de raclage (39, 339) adaptée à la section droite de la saillie (24, 124, 224, 324) pour de la matière de constitution d'échantillon (68) adhérant à la périphérie de la saillie (24, 124, 224, 324), et en ce qu'une cavité annulaire (34, 134, 234, 334) se fermant vers l'extérieur, destinée à recevoir la matière de constitution d'échantillon (68) retirée, est formée dans l'élément formant couvercle (14, 114, 214, 314) et/ou dans la section de récipient, du côté de la collerette de raclage (39) éloigné de la cavité intérieure (18, 118, 218, 318) du récipient.

2. Récipient selon la revendication 1, caractérisé en ce que l'élément formant couvercle (14, 114, 214, 314) et la section de récipient (12, 112, 212, 312) comportent un joint périphérique isolant hermétiquement la cavité annulaire (34, 134, 234, 334) de l'extérieur, de préférence en forme d'un collet périphérique (29, 329) en saillie sur la face intérieure de l'élément formant couvercle, qui est en contact étanche, par une surface périphérique intérieure, avec une surface périphérique extérieure d'un épaulement périphérique de la section de récipient (12, 112, 212, 312).

3. Récipient selon l'une quelconque des revendications 1 ou 2, caractérisé en ce que la saillie (24, 124, 224, 324) dépasse du collet périphérique (29, 329), de préférence de 1 à 5 mm, au mieux d'environ 2 mm.

4. Récipient selon l'une quelconque des revendications précédentes, caractérisé en ce que la section de récipient (12, 112) comporte une autre ouverture en liaison avec la cavité intérieure (18, 118) du récipient, qui est fermée par un élément formant couvercle supplémentaire amovible (16, 116).

5. Récipient selon la revendication 4, caractérisé en ce que la section de récipient (12, 112) est tubulaire et est fermée à l'une des extrémités du tube qui la constitue par l'élément formant couvercle (14, 114) et, à l'autre extrémité de ce tube, par l'élément formant couvercle supplémentaire (16, 116).

6. Récipient selon l'une quelconque des revendications précédentes, caractérisé en ce que l'élément formant couvercle (14, 114, 214, 314) et/ou l'élément formant couvercle supplémentaire (16, 116) sont reliés chacun, de préférence d'un seul tenant, à la section de récipient (12, 112, 312) par une languette de liaison respective (54; 56).

7. Récipient selon l'une quelconque des revendications précédentes, caractérisé en ce que l'élément formant couvercle (14, 114, 214, 314) et/ou l'élément formant couvercle supplémentaire (16, 116) sont reliés, de préférence d'un seul tenant, à une languette de prise (58, 59, 258).

8. Récipient selon l'une quelconque des revendications précédentes, caractérisé en ce que la section de récipient (12) et l'élément formant couvercle (14) et/ou l'élément formant couvercle supplémentaire (16) présentent un diamètre extérieur (i) d'environ 17 mm, et en ce que les languettes de liaison et de prise (54, 56, 58, 59) sont éventuellement facilement séparables, de préférence grâce à des points destinés à la rupture (70).

9. Récipient selon l'une quelconque des revendications précédentes, caractérisé en ce que la surface intérieure (44) de la section de récipient délimitant la cavité intérieure (18, 118) du récipient s'amincit de préférence en cône en direction de l'évidement de réception d'échantillon (42, 142) de l'élément formant couvercle (14, 114).

10. Récipient selon l'une quelconque des revendications précédentes, caractérisé en ce que la section de récipient (12; 112) comporte un collet périphérique intérieur (40, 140, 340), de préférence contre-dépouillé sur la face frontale (43; 143) de la saillie (24; 124; 324) dans la zone du bord de l'évidement de réception d'échantillon (42, 142, 342).

11. Récipient selon l'une quelconque des revendications précédentes, caractérisé par une baguette d'homogénéisation (62) de conformation en spirale pouvant être introduite dans la cavité intérieure (18, 118) du récipient, dont le diamètre (h) de la spirale est adapté au diamètre (d) de la cavité intérieure du récipient.

12. Récipient selon la revendication 11, caractérisé en ce que l'extrémité (64), proche de l'évidement de réception d'échantillon (42, 142), de la baguette d'homogénéisation (62), s'étend sensiblement dans la direction de l'axe (63) de la spirale, de préférence à distance de l'axe (63) de la spirale.

13. Récipient selon l'une quelconque des revendications précédentes, caractérisé en ce que la saillie (324) s'amincit légèrement en cône en direction de l'évidement de réception d'échantillon (342).

14. Récipient selon l'une quelconque des revendications précédentes, caractérisé par une cavité de réception (38, 338) pour la saillie (24, 324) dans la section de récipient (12, 312), à l'extrémité d'insertion de saillie de laquelle se trouve la collerette de raclage (39, 339) et à l'extrémité opposée, se raccordant avec la cavité intérieure (18, 318) du récipient, de laquelle se trouve éventuellement le collet périphérique intérieur contre-dépouillé.

15. Récipient selon la revendication 14, caractérisé en ce que la cavité de réception (38, 338) a une conformation cylindrique ou s'amincissant légèrement en cône vers la cavité intérieure du récipient.

16. Récipient selon l'une quelconque des revendications 2 à 15, caractérisé en ce que la collerette périphérique (326) peut être fixée à l'épaulement périphérique de la section de récipient (312) au moyen d'un assemblage à enclenchement, conformé, de préférence, avec au moins une protubérance périphérique (380) prévue sur l'un des deux éléments, qui pénètre dans un creux périphérique (382) prévu dans l'autre élément respectif.

17. Application du récipient selon l'une quelconque des revendications précédentes au dosage d'échantillon de selles de quantités inférieures à 1 g de selles, de préférence comprises entre 20 et 200 mg de selles, en particulier pour la détermination de l'activité des enzymes, de préférence la détermination de la chymotrypsine ou de la trypsine, dans les selles.

18. Application du récipient selon la revendication 17 au transport d'échantillons, notamment à l'expédition d'échantillons.

19. Application du récipient selon l'une des revendications 17 et 18 à l'homogénéisation d'échantillons dans la cavité intérieure du récipient, de préférence en retirant le couvercle supplémentaire, en ajoutant un agent d'homogénéisation et des éléments d'homogénéisation, de préférence la baguette d'homogénéisation, en appliquant le couvercle supplémentaire et en secouant ensuite le récipient dans un vibrateur à secousses.

20. Application du récipient selon l'une quelconque des revendications 17 à 19 à la centrifugation en retirant les languettes de liaison et les languettes de prise, puis en introduisant le récipient dans une centrifugeuse.

FIG. 1

FIG. 2

0 076 507

FIG. 3

FIG. 4

0 076 507

FIG.7

FIG. 5

FIG. 6

0 076 507

0 076 507

21